Europäisches Patentamt

European Patent Office

Office européen des brevets

(11) Veröffentlichungsnummer : **0 107 051 B1**

(12) **EUROPÄISCHE PATENTSCHRIFT**

(45) Veröffentlichungstag der Patentschrift :
30.12.86

(21) Anmeldenummer : 83109469.3

(22) Anmeldetag : 23.09.83

(51) Int. Cl.⁴ : **A 61 F 13/00**

(54) **Wundauflage für Verbände oder Kompressen.**

(30) Priorität : 30.09.82 JP 172930/82

(43) Veröffentlichungstag der Anmeldung :
02.05.84 Patentblatt 84/18

(45) Bekanntmachung des Hinweises auf die Patenterteilung : 30.12.86 Patentblatt 86/52

(84) Benannte Vertragsstaaten :
**DE FR GB IT SE**

(56) Entgegenhaltungen :
CH-A-    523 366
DE-C-    977 543
GB-A- 2 024 709
GB-A- 2 055 690
US-A- 3 171 773

(73) Patentinhaber : **Firma Carl Freudenberg
Höhnerweg 4
D-6940 Weinheim/Bergstrasse (DE)**

(72) Erfinder : **Kimura, Hideo
900-67 Nishi Ushigaya Oaza, Sohwa-machi
Sashima-gun Ibaraki-ken (JP)**
Erfinder : **Tabuchi, Masahiro
1391 Komahane Sohwa-machi
Sashima-gun Ibaraki-ken (JP)**
Erfinder : **Iwasaki, Motokazu
1399 Komahane Sohwa-machi
Sashima-gun Ibaraki-ken (JP)**

(74) Vertreter : **Weissenfeld-Richters, Helga, Dr.
Höhnerweg 2
D-6940 Weinheim/Bergstrasse (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft eine Wundauflage aus Vliesstoff für Verbände oder Kompressen zum Aufbringen medizinischer Wirkstoffe auf die Haut.

Wundauflagen dieser Art sind aus CH-A-523 366 bekannt und weisen gegenüber vergleichbaren Produkten aus Geweben oder Gewirken Vorteile auf.

Insbesondere hat sich ihre gute Flexibilität und Luftdurchlässigkeit bewährt. Die Flexibilität ermöglicht eine verbesserte Anpassung an die zu behandelnde Hautoberfläche und die gute Luftdurchlässigkeit fördert die Wundheilung. Mit der an sich als vorteilhaft empfundenen Luftdurchlässigkeit ist aber der schwerwiegende Nachteil verbunden, daß medizinische Wirkstoffe in die Vliesstoffauflage eindringen und mehr oder weniger schnell in die hautabgewandte Seite der Auflage durchbluten. Das Durchbluten kann zwar durch eine dickere Vliesstoffschicht vermindert werden, jedoch führt dies zu einem höheren Preis der Auflage und einer verminderten Flexibilität sowie zu einer Abschwächung der Wirksamkeit aufgetragener Medikamente, weil diese im wesentlichen in das Innere der Wundauflage eindringen und somit auf der Hautoberfläche nicht mehr zur Verfügung stehen.

Zur Vermeidung bzw. Verminderung des Durchblutens ist es auch schon versucht worden, die Vliesstoff-Wundauflage an der hautabgewandten Seite mit undruchlässigen Flächengebilden abzudecken, jedoch leidet hierdurch wiederum die Luftdurchlässigkeit und die Wundheilung wird in unerwünschtem Maße verzögert.

Der Erfindung liegt die Aufgabe zugrunde, eine leichte und flexible Wundauflage aus Vliesstoff zu entwickeln, die das Aufbringen medizinischer Wirkstoffe auf die Haut in Form von Verbänden oder Kompressen ermöglicht, ohne daß die Wirkstoffe in unerwünschtem Maße in die Kompresse hineindiffundieren bzw. auf die entgegengesetzte Seite ausbluten. Die Wundauflage soll weiterhin für solche medizinischen Wirkstoffe geeignet sein, die über einen längeren Zeitraum hinweg ohne Wechsel der Auflage auf die Hautoberfläche einwirken sollen.

Die erfindungsgemäß gestellte Aufgabe wird durch die in den Patentansprüchen beschriebene Wundauflage gelöst.

Der medizinische Wirkstoff wird auf die Haut bzw. einseitig auf die Wundauflage aufgetragen, wobei die dem medizinischen Wirkstoff bzw. der Hautoberfläche zugewandte Seite der Wundauflage erfindungsgemäß wenigstens 20 Gew.-%, bezogen auf deren Gesamtgewicht, an Fasern und/oder Filamenten mit abgeflachtem Querschnitt besteht. Übliche Fasern oder Filamente haben einem im wesentlichen kreisförmigen Querschnitt. Die abgeflachten Fasern oder Filamente können in einfacher Weise durch Ausspinnen mit Hilfe elliptischer, C-förmiger oder rechteckiger Düsen erhalten werden, wobei die Spinnfäden einen der Düsenform entsprechenden Querschnitt haben. Aus derartigen Filamenten bzw. aus Fasern, die durch Zerkleinern derartiger Filamente gewonnen wurden, wird die Wundauflage hergestellt. Die Vliesstoff-Wundauflage kann bis zu 100 Gew.-% abgeflachte Fasern und/oder Filamente enthalten. Sie muß wenigstens 20 Gew.-% derartiger Fasern wenigstens an der hautzugewandten Seite enthalten, wobei die restlichen Fasern solche mit im wesentlichen kreisförmigem Querschnitt sind. Abgeflachte Fasern können auch durch elektrostatisches Versprühen hergestellt werden. Derartige Fasern weisen in der Regel einen hantelförmigen Querschnitt auf.

Der abgeflachte Faserquerschnitt ist so beschaffen, daß das Verhältnis der großen zur kleinen Achse wenigstens 2 : 1 beträgt. Innerhalb des Vliesstoffes sind die abgeflachten Fasern dann so angeordnet, daß die großen Achsen der Faser- bzw. Filamentquerschnitte zu der ebenen Oberfläche der Wundauflage einen Winkel zwischen 0° und 75° bilden.

Größere Winkel als 75° bringen stark verschlechterte Ergebnisse im Hinblick auf das Durchbluten der medizinischen Wirkstoffe. Insbesondere Winkel im Bereich von 90° ergeben sehr unbefriedigende Resultate.

Falls weniger als 100 Gew.-% abgeflachte Fasern in dem Vliesstoff der Wundauflage vorhanden sind, können übliche Fasern mit im wesentlichen kreisförmigem Querschnitt beigemischt sein. Es können dies geeignete Fasern und/oder Filamente aus natürlichen oder synthetischen Rohstoffen sein.

Die abgeflachten Fasern verhindern das Durchbluten der medizinischen Wirkstoffe auf die der Haut abgewandte Seite. Durch die Abflachung des Querschnitts und die spezielle Anordnung der Fasern bzw. Filamente in dem angegebenen Winkel zur ebenen Oberfläche der Wundauflage kann das Durchbluten gezielt verhindert bzw. vermindert werden, wobei die Luftdurchlässigkeit der Auflage erhalten bleibt. Besonders bewährt haben sich abgeflachte Fasern mit C-förmigem oder elliptischem Querschnitt.

Die abgeflachten Fasern werden in einer Stärke von 1 bis 10 Denier verwendet. Es ist zweckmäßig, wenn auch die nicht abgeflachten Fasern innerhalb dieses Bereiches liegen.

Zweckmäßig bestehen die abgeflachten Fasern und/oder Filamente aus Polypropylen, Polyethylen, Polyamid, Polyester oder Zellulose.

Unter « großer » bzw. « kleiner » Achse des Faserquerschnitts werden z. B. bei elliptischem Querschnitt die üblicherweise als große bzw. kleine Achse bekannte Bezeichnung verstanden.

Bei C-förmigem oder rinnenförmigem Querschnitt mit dreieckigem Profil wird der Abstand zwischen den Endpunkten des C oder die Basisfläche des Dreiecks als « große » Achse verstanden und die Dicke der Faser als « kleine » Achse. Bei allen « abgeflachten » Querschnitten soll das Achsenverhältnis wenigstens 2 : 1 betragen.

Die Wundauflage ist ein- oder mehrlagig aufgebaut. Bei einlagigem Aufbau besteht die gesamte Wundauflage aus dem beschriebenen Vliesstoff mit wenigstens 20 Gew.-% abgeflachten Fasern. Bei mehrlagigem Aufbau muß wenigstens die der Haut zugewandte Lage den beschriebenen Aufbau aufweisen, während weitere hautabgewandte Lagen aus dem erfindungsgemäßen oder üblichen Vliesstoffen vorgesehen sein können. Das Durchbluten der medizinischen Wirkstoffe wird in jedem Falle durch die Schicht mit dem erfindungsgemäßen Aufbau verhindert.

Der Winkel der großen Achsen der Faser- bzw. Filamentquerschnitte muß zu der ebenen Oberfläche der Wundauflage Werte zwischen 0 und 75° bilden. Bei einem Winkel von 0° liegen die Fasern bzw. Filamente zur Wundauflagenoberfläche parallel und bilden einen nahezu vollständigen Abschluß. Bei größeren Winkeln bis zu 75° ergibt sich eine mehr oder weniger große Porosität, welche die erwünschte Luftzirkulation gewährleistet und die Heilung der Wunde fördert, jedoch ist das Durchbluten der Wirkstoffe infolge der abgeflachten Querschnitte der Fasern verhindert.

Es ist wichtig, daß nahezu alle Fasern und Filamente in dem beschriebenen Sinne ausgerichtet sind. Die Abweichung von dieser Ausrichtung soll, wenn überhaupt, so nur zu einem geringen Prozentsatz vorhanden sein.

Die Ausrichtung kann in einfacher Weise durch übliche Karden oder Krempelmaschinen erreicht werden. Der mit Hilfe derartiger Karden oder Krempelmaschinen erhaltene Flor wird zweckmäßig mit Hilfe von Wärme und Druck leicht an den Faserkreuzungspunkten gebunden und gegebenenfalls genadelt. Auf diese Weise werden die abgeflachten Fasern in der gewünschten Richtung festgehalten. Der Vliesstoff wird nun zugeschnitten und kann in dieser Form mit dem Medikament beaufschlagt werden. Er kann jedoch auch ohne Medikament, das dann direkt auf die Haut aufgetragen wird, Anwendung finden.

Die Wundauflage dient im wesentlichen zur Herstellung von Verbänden oder Kompressen. Sie kann jedoch auch als Auflage für Wundpflaster und zu Spezialverbänden, z. B. Brandbinden oder dergl. verarbeitet werden.

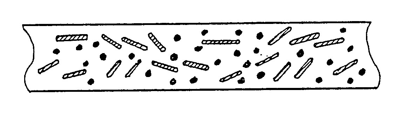

Figur 1 zeigt einen Querschnitt durch die erfindungsgemäße Wundauflage. Die großen Achsen der abgeflachten Fasern bilden zu der ebenen Oberfläche der Auflage Winkel zwischen 0 und 75°. Dazwischen sind Fasern mit kreisförmigem Querschnitt angeordnet.

Die nachfolgenden Beispiele dienen zur Erläuterung der Erfindung.

## Beispiel 1

50 Gew.-% flacher Rayonfasern von 5 Denier mit einem Achsenverhältnis von 20 : 1 werden mit 50 Gew.-% 3 Denier starker Fasern aus Polypropylen mit einem Mantel aus Polyethylen vermischt. Die Fasern werden mit Hilfe einer Karde zu einem Flor abgelegt und bei 140 °C innerhalb von 30 Sekunden durch Kalandrieren verfestigt. Hierbei erweichen die Polyethylenmäntel der zusammengesetzten Fasern und bewirken ein Verkleben der Fasern der Vlieses an den Überkreuzungsstellen. Der so erhaltene Vliesstoff ist 0,5 mm dick und weist ein Gewicht von 60 g/m² auf.

Auf diesen Vliesstoff wird einseitig ein Medikament zur äußerlichen Anwendung aufgetragen, das aus 45 Gewichtsteilen Kaolin, 30 Gewichtsteilen destilliertem Wasser, 20 Gewichtsteilen Glyzerin und 10 Gewichtsteilen Polyvinylalkohol als wesentliche Bestandteile besteht. Die restlichen Gewichtsteile bestehen aus Salizylsäuremethylester, Pfefferminzöl, Menthol oder dergleichen. Der Wirkstoff wird mit einem abziehbaren Film abgedeckt. Die Wundauflage kommt in dieser Form zum Versand. Bei Gebrauch wird der abziehbare Film entfernt.

Die so hergestellte Wundauflage zeigt auch nach mehr als 20tägigem Verbrauch kein Durchbluten der medizinischen Wirkstoffe.

Zum Vergleich werden nachfolgend drei nicht erfindungsgemäß aufgebaute Wundauflagen beschrieben.

## Auflage A

50 Gew.-% 3 Denier Rayonfasern mit querförmigem Querschnitt werden mit 50 Gew.-% der gleichen zusammengesetzten Faser wie in Beispiel 1 vermischt. Die Mischung wird in gleicher Weise, wie in Beispiel 1 beschrieben, verarbeitet und in gleicher Weise mit dem gleichen Medikament beschichtet. Der Vliesstoff ist 0,63 mm dick und weist ein Gewicht von 60 g/m² auf.

## Auflage B

Es wird ein Vliesstoff hergestellt, der hinsichtlich des Aufbaues der Auflage A entspricht, jedoch eine Dicke von 0,85 mm und ein Gewicht von 90 g/m² aufweist.

## Auflage C

Ein Vliesstoff entsprechend der Auflage A wird zur Verminderung des Durchblutens oberflächlich versiegelt. Hierzu wird eine Mischung aus 80 Gew.-% Kaolin, 10 Gew.-% Polyvinylalkohol und 10 Gew.-%

Polyacrylsäureester verwendet. Diese Mischung wird in einer Menge von 40 g/m² aufgetragen. Es wird dann der gleiche medizinische Wirkstoff wie in Beispiel 1 auf der hautzugewandten Seite der Wundauflage aufgetragen.

Die nach Beispiel 1 hergestellte erfindungsgemäße Wundauflage sowie die Auflagen A, B und C werden bei der Durchführung des Tests unter einem Druck von 1 kg/10 cm. 15 cm auf eine ebene Fläche gepreßt und bei einer Temperatur von 40 °C gehalten. Das Durchbluten wird von Zeit zu Zeit getestet. wobei jeweils 5 Probeexemplare jeder Auflage untersucht werden. Unter « Durchbluten » wird das Durchdringen des weichen Wirkstoffes an die Oberfläche der Auflage verstanden. Die Testergebnisse sind in der nachfolgenden Tabelle wiedergegeben.

| | 5 Tage | 10 Tage | 15 Tage | 20 Tage |
|---|---|---|---|---|
| Wundauflage nach Beispiel 1 | - | - | - | - |
| Auflage A | + | ++ | ++ | ++ |
| Auflage B | - | - | ± | + |
| Auflage C | - | - | - | ± |

Die in der Tabelle verwendeten Symbole haben folgende Bedeutung :
— : Bei allen fünf Testauflagen wird kein Durchbluten beobachtet.
± : Bei wenigstens einer der fünf Testauflagen wird leichtes Durchbluten beobachtet.
+ : Bei allen fünf Auflagen wird Durchbluten beobachtet.
++ : Bei allen fünf Auflagen wird ein Durchbluten zu wenigstens 20 % der Oberfläche beobachtet.

Beispiel 2

50 Gew.-% einer abgeflachten Nylonfaser von 1,5 Denier mit einem Achsenverhältnis von 2,5 : 1 wird mit 30 Gew.-% 3 Denier Polyesterfasern mit kreisförmigem Querschnitt und 20 Gew.-% 3 Denier Rayonfasern mit kreisförmigem Querschnitt vermischt. Die Mischung wird mit Hilfe einer Karde zu einem Flor von 40 g/m² abgelegt. Der Flor wird mit einem Bindemittel auf Polyacrylesterbasis gebunden, das in Form einer Emulsion angewandt wird, mit dem der Flor imprägniert ist. Nach dem Trocknen wird ein Vliesstoff von 60 g/m² erhalten. Dieser Vliesstoff dient als Wundauflage, mit dem das gleiche Medikament, wie in Beispiel 1 beschriebene, beschichtet wird. Jeweils 5 Testauflagen werden in gleicher Weise wie in Beispiel 1 beschrieben getestet und bei allen fünf Auflagen wird auch nach 20 Tagen kein Durchbluten beobachtet.

Beispiel 3

60 Gew.-% abgeflachter Rayonfasern von 5 Denier mit einem Achsenverhältnis von 20 : 1 und 40 Gew.-% 3 Denier Polyesterfasern mit kreisförmigem Querschnitt werden vermischt. Die Mischung wird mit Hilfe einer Karde zu einem Vlies von 12 g/m² abgelegt. Als Bindemittel dient eine Polyacrylesteremulsion. mit der der Flor imprägniert wird. Nach dem Trocken erhält man einen Vliesstoff von 15 g/m². Dieser Vliesstof wird mit einem Vlies laminiert, das aus 3 Denier Polyesterfasern mit querförmigem Querschnitt besteht. Das Laminiervlies hat ein Gewicht von 85 g/m². Das Laminat wird dann genadelt. Der so erhaltene zweischichtige Vliesstoff besteht aus einer Lage mit abgeflachten Fasern und einer weiteren Lage mit üblichen Fasern, die einen im wesentlichen kreisförmigen Querschnitt aufweisen. Auf der Seite, an der sich das Vlies mit abgeflachten Fasern befindet, wird der in Beispiel 1 beschriebene Wirkstoff aufgetragen. Der in gleicher Weise wie in Beispiel 1 durchgeführte Testversuch ergab bei allen fünf Testauflagen selbst nach vier Wochen kein Durchbluten.

## 0 107 051

### Vergleichsbeispiele

Es werden Rayonfasern von 5 Denier mit kreisförmigem Querschnitt anstelle der abgeflachten Fasern in Beispiel 3 verwendet. Im übrigen wird ein identischer zweilagiger Vliesstoff, wie in Beispiel 3 beschrieben, hergestellt. Die aus diesem Vliesstoff geschnittene Wundauflage wird mit dem gleichen Wirkstoff beschichtet.

Der Testversuch gemäß Beispiel 1 ergibt bei allen fünf Testauflagen ein Durchbluten nach zwei Wochen ab Beginn.

### Patentansprüche

1. Ein- oder mehrlagige Wundauflage aus Vliesstoff für Verbände oder Kompressen zum Aufbringen medizinischer Wirkstoffe auf die Haut, dadurch gekennzeichnet, daß der Vliesstoff wenigstens an der hautzugewandten Seite aus mindestens 20 Gew.-%, bezogen auf deren Gesamtgewicht, aus Fasern und/oder Filamenten mit abgeflachtem Querschnitt besteht, daß das Verhältnis der großen zur kleinen Achse der Faserquerschnitte wenigstens 2 : 1 beträgt, und daß die Fasern und/oder Filamente in dem Vliesstoff wenigstens dieser Schicht so angeordnet sind, daß die großen Achsen der Faser- bzw. Filamentquerschnitte zu der ebenen Oberfläche der Wundauflage einen Winkel zwischen 0° and 75° bilden.

2. Wundauflage nach Anspruch 1, dadurch gekennzeichnet, daß die Fasern und/oder Filamente eine Stärke von 1 bis 10 Denier aufweisen.

3. Wundauflage nach einem der Ansprüche 1 oder 2, dadurch gekennzeichnet, daß wenigstens die Fasern und/oder Filamente mit abgeflachtem Querschnitt eine Stärke von 1 bis 10 Denier aufweisen.

4. Wundauflage nach einem der Ansprüche 1 bis 3, dadurch gekennzeichnet, daß die abgeflachten Fasern und/oder Filamente einen C-förmigen oder elliptischen Querschnitt aufweisen.

5. Wundauflage nach einem der Ansprüche 1 bis 4, dadurch gekennzeichnet, daß die abgeflachten Fasern und/oder Filamente aus Polypropylen, Polyethylen, Polyamid, Polyester oder Zellulose bestehen.

6. Wundauflage nach einem der Ansprüche 1 bis 5, dadurch gekennzeichnet, daß die abgeflachten Fasern und/oder Filamente zusammen mit nicht abgeflachten Fasern und/oder Filamenten mit im wesentlichen kreisförmigem Querschnitt und aus natürlichen und/oder synthetischen Rohstoffen verwendet sind.

### Claims

1. A uni-layered or multi-layered nonwoven wound covering for bandages or compresses for applying medicinally active substances to the skin, characterised in that at least on the side facing the skin the nonwoven material contains at least 20 % by weight, relative to its total weight, of fibres and/or filaments having a flattened cross-section, that the ratio of the major axis to the minor axis of the flattened cross-section is at least 2 : 1, in that the fibres and/or filaments of at least this layer of the nonwoven material have been arranged in such a way that the major axis of the flattened cross-section of the fibres and/or filaments is at an angle of from 0° to 75° to the plane surface of the wound covering.

2. A wound covering according to claim 1, characterised in that the fibres and/or filaments have a thickness of 1 to 10 denier.

3. A wound covering according to claim 1 or 2, characterised in that at least the fibres and/or filaments having a flattened cross-section have a thickness of 1 to 10 denier.

4. A wound covering according to any of claims 1 to 3, characterised in that the flattened fibres and/or filaments have a C-shaped or elliptical cross-section.

5. A wound covering according to any of claims 1 to 4, characterised in that the flattened fibres and/or filaments are formed of polypropylene, polyethylene, polyamide, polyester or cellulose.

6. A wound covering according to any of claims 1 to 5, characterised in that the flattened fibres and/or filaments have been used together with non-flattened fibres and/or filaments having an essentially circular cross-section and formed of natural and/or synthetic raw materials.

### Revendications

1. Recouvrement de plaies à une ou plusieurs couches constitué d'un voile pour bandages ou compresses en vue d'appliquer des substances actives médicamenteuses sur la peau, caractérisé en ce que, au moins sur la face tournée vers la peau, le voile est constitué d'au moins 20 % en poids (calculé sur le poids total) de fibres et/ou de filaments à section transversale aplatie, le rapport entre le grand et le petit axe de la section transversale des fibres étant d'au moins 2 : 1, tandis que les fibres et/ou les filaments du voile sont disposés au moins dans cette couche de telle sorte que les grands axes des sections transversales des fibres ou des filaments par rapport à la surface plane du recouvrement de plaies

5

forment un angle compris entre 0 et 75°.

2. Recouvrement de plaies selon la revendication 1, caractérisé en ce que les fibres et/ou les filaments ont une épaisseur de 1 à 10 deniers.

3. Recouvrement de plaies selon l'une des revendications 1 ou 2, caractérisé en ce qu'au moins les fibres et/ou les filaments à section transversale aplatie ont une épaisseur de 1 à 10 deniers.

4. Recouvrement de plaies selon une des revendications 1 à 3, caractérisé en ce que les fibres et/ou les filaments aplatis ont une section transversale en C ou elliptique.

5. Recouvrement de plaies selon une des revendications 1 à 4, caractérisé en ce que les fibres et/ou les filaments aplatis sont constitués de polypropylène, de polyéthylène, de polyamide, de polyester ou de cellulose.

6. Recouvrement de plaies selon une des revendications 1 à 5, caractérisé en ce qu'on utilise les fibres et/ou les filaments aplatis conjointement avec des fibres et/ou des filaments non aplatis à section transversale essentiellement circulaire et constitués de matières premières naturelles et/ou synthétiques.